# FASCICULE DE BREVET EUROPEEN

(11) **EP 3 149 498 B1**
(45) Date de publication et mention de la délivrance du brevet: **09.10.2019**
(21) Numéro de dépôt: 15725615.7
(22) Date de dépôt: 27.05.2015
(51) Int. Cl.: G01R 27/26, G01N 22/00

(54) **ROCEDE D'AMELIORATION DE L'HOMOGENEITE ET DE L'INTENSITE D'UN CHAMP ELECTRIQUE INDUIT DANS UN OBJET ILLUMINE PAR UNE ONDE ELECTROMAGNETIQUE INCIDENTE**
VERFAHREN ZUR VERBESSERUNG DER GLEICHFÖRMIGKEIT UND INTENSITÄT EINES ELEKTRISCHEN FELDES IN EINEM DURCH EINE EINFALLENDE ELEKTROMAGNETISCHE WELLE BELEUCHTETEN OBJEKT
METHOD FOR IMPROVING THE UNIFORMITY AND INTENSITY OF AN ELECTRIC FIELD IN AN OBJECT ILLUMINATED BY AN INCIDENT ELECTROMAGNETIC WAVE

(30) Priorité: 28.05.2014 FR 1454821
(43) Date de publication de la demande: 05.04.2017
(73) Titulaire: Commissariat à l'Énergie Atomique et aux Énergies Alternatives, 75015 Paris (FR)
(72) Inventeur: VEZINET, René, F-46500 Bio (FR); CATRAIN, Alexandre, F-46300 Le Vigan (FR); CHRETIENNOT, Thomas, F-46500 Gramat (FR)
(74) Mandataire: Brevalex
(86) Numéro de dépôt international: PCT/EP2015/061758
(87) Numéro de publication internationale: WO 2015/181258

(56) Documents cités:
- US-A- 5 084 910
- US-A1- 2001 050 810
- L. LAVAL ET AL: "A new in vitro exposure device for the mobile frequency of 900 MHz", BIOELECTROMAGNETICS, vol. 21, no. 4, 1 mai 2000 (2000-05-01), pages 255-263, XP055164614, ISSN: 0197-8462, DOI: 10.1002/(SICI)1521-186X(200005)21:4<255::A ID-BEM2>3.0.CO;2-4
- SCHUDERER J ET AL: "IN VITRO EXPOSURE SYSTEMS FOR RF EXPOSURES AT 900 MHZ", IEEE TRANSACTIONS ON MICROWAVE THEORY AND TECHNIQUES, IEEE SERVICE CENTER, PISCATAWAY, NJ, US, vol. 52, no. 8, PART 02, 1 août 2004 (2004-08-01), pages 2067-2075, XP001200626, ISSN: 0018-9480, DOI: 10.1109/TMTT.2004.832010
- L. D. ANGULO ET AL: "Improving the SAR Distribution in Petri-Dish Cell Cultures", JOURNAL OF ELECTROMAGNETIC WAVES AND APPLICATIONS, vol. 24, no. 5-6, 1 janvier 2010 (2010-01-01), pages 815-826, XP055164688, ISSN: 0920-5071, DOI: 10.1163/156939310791036322
- FONTANA N ET AL: "On the influence of a glass slide on the SAR distribution in Petri dishes for in vitro exposure to 2.45 GHz EM fields", ANTENNAS AND PROPAGATION SOCIETY INTERNATIONAL SYMPOSIUM (APSURSI), 2010 IEEE, IEEE, PISCATAWAY, NJ, USA, 11 juillet 2010 (2010-07-11), pages 1-4, XP032145600, DOI: 10.1109/APS.2010.5561218 ISBN: 978-1-4244-4967-5

## Description

### DOMAINE TECHNIQUE

Le domaine de l'invention est celui de l'électromagnétisme et du bio-électromagnétisme. L'invention trouve en particulier application dans le domaine de la caractérisation dosimétrique d'un objet à tester lorsque ce dernier est illuminé par une onde électromagnétique incidente en espace libre ou dans un système d'exposition à des fins expérimentales, pour l'étude des interactions des ondes électromagnétiques avec le vivant.

### ÉTAT DE LA TECHNIQUE ANTÉRIEURE

Les études conduites dans le domaine de la bioélectricité et, par extension, du bio-électromagnétisme, ont pour but d'étudier les effets des champs électromagnétiques sur le vivant. Ces études nécessitent des systèmes expérimentaux permettant d'exposer aux champs électromagnétiques divers organismes biologiques ou chimiques, de tailles et de natures variées, allant de la molécule ou de la cellule jusqu'à des organismes complets (plantes, souris, etc.).

Il peut s'agir d'études à finalité normative portant sur la détermination des seuils de nocivité et d'innocuité (téléphonie, transmissions, applications électromagnétiques de défense, appareillages électriques, etc.), à finalité médicale (traitement du cancer, stimulation neuronale, etc.) ou bien encore à finalité industrielle (décontamination, stérilisation, processus de transformation des aliments, etc.).

Ces études expérimentales mettent en oeuvre des systèmes d'exposition qui doivent assurer la fonction de support pour les objets sous test durant les phases d'illumination par un champ électromagnétique ; généralement, ils doivent aussi assurer la fonction de contenant, car les objets à tester sont le plus souvent sous la forme d'un liquide. On peut par exemple s'intéresser à l'exposition d'objets biologiques de tailles microscopiques, telles que des cellules, des GUV (Vésicules Uni lamellaires Géantes) ou des sphéroïdes, qui sont généralement en suspension dans un milieu biologique (solution à faible résistivité électrique). Les contenants sont le plus souvent de formes cylindriques, qu'il s'agisse de tubes Eppendorf™, de boîtes de Petri ou autres.

On utilise actuellement de nombreux systèmes d'exposition expérimentaux, tels que les systèmes d'exposition type « onde plane », les chambres réverbérantes (CRBM), les guides d'onde (cylindriques ou rectangulaires), les cellules fil-plaque, les lignes de transmission radiale ou encore les lignes de transmission à conducteurs plans, comme par exemple les cellules TEM (Transverse Electro Magnétique).

L'inconvénient de ces systèmes est que l'homogénéité du champ électromagnétique incident est plus ou moins bonne selon les systèmes d'exposition utilisés.

D'autre part, même dans le cas idéal d'une exposition en onde plane (illumination par une antenne lointaine en espace libre) pour lequel l'homogénéité du champ incident est parfaite, il s'avère que l'homogénéité et l'intensité du champ électrique induit dans le volume de l'objet sous test dépendent de la nature et de la géométrie de l'objet sous test, ainsi que de la polarisation et de l'angle d'incidence de l'onde incidente.

Or, il est primordial d'avoir une très bonne homogénéité du champ dans le volume de l'OST et d'avoir un bon facteur de couplage (intensité du champ induit/champ incident) et d'avoir une parfaite maîtrise des champs pénétrant les volumes des objets sous test afin d'assurer la validation et la reproductibilité des expériences.

L. Laval et al, "A New In Vitro Exposure Device for the Mobile Frequency of 900MHz", Bioelectromechanics 21:255-263 (2000) décrit un système où une boîte de Pétri est placé dans une boîte de Petri plus grande et rempli d'eau afin d'améliorer l'homogénéité du champs dans le volume a tester. Cependant, le paroi de la boîte de Petri en contact avec l'objet à tester introduit un degré d'inhomogénéité.

### EXPOSÉ DE L'INVENTION

L'invention a pour objectif principal d'améliorer l'homogénéité et d'augmenter l'intensité d'un champ électrique induit dans un objet sous test soumis à un champ électrique incident.

A cet égard, l'invention propose un procédé d'amélioration de l'homogénéité et de l'intensité du champ électrique selon les revendications 1 ou 2 comprenant une étape de préparation d'un objet à tester destiné à être illuminé par une onde électromagnétique incidente, l'objet présentant une permittivité relative donnée, lequel procédé comprend :
- la fourniture d'une pièce comprenant une cavité pour le logement de l'objet et au moins un élément d'extension en un matériau présentant une permittivité relative égale à la permittivité relative de l'objet à +/- 50% près, ledit au moins un élément d'extension délimitant en partie la cavité et s'étendant de part et d'autre de la cavité selon une direction qui est dite direction de traversée de la cavité, sur une longueur qui est au moins égale, de part et d'autre de la cavité, au tiers de la longueur de la cavité selon la direction de traversée ; et
- la mise en place de l'objet dans la cavité, de manière à ce que l'objet soit en contact avec ledit au moins un élément d'extension selon la direction de traversée.

Avantageusement, un élément d'extension a une permittivité relative égale à la permittivité relative de l'objet à +/-40 % près, de préférence à +/- 30 % près, de préférence encore à +/- 25% près, préférentiellement à +/- 20% près, et encore plus préférentiellement à +/- 10% près. De préférence, s'il y a plusieurs éléments d'extension, on choisit des éléments d'extension ayant la même permittivité relative. En fait, l'efficacité du procédé objet de l'invention est maximale lorsque la permittivité relative du ou des éléments d'extension est égale à celle de l'objet et se dégrade avec l'écart entre les permittivités relatives de l'objet et du ou des éléments d'extension.

De préférence, un élément d'extension a une permittivité relative égale à la permittivité relative de l'objet.

Selon un mode de réalisation préféré, l'objet à tester a une conductivité électrique donnée et ledit au moins un élément d'extension a une conductivité électrique égale à celle de l'objet à +/- 30% près, de préférence à +/- 25% près, préférentiellement à +/- 20% près, et encore plus préférentiellement à +/- 10% près. Il est à noter qu'il est tout à fait possible d'utiliser un ou plusieurs éléments d'extension ayant un matériau avec une conductivité électrique nulle ou très faible. Toutefois, en choisissant un matériau d'extension avec une conductivité électrique proche, idéalement égale, à celle de l'objet, on améliore l'efficacité du procédé. De préférence, s'il y a plusieurs éléments d'extension, on choisit des éléments d'extension en un même matériau, afin qu'ils aient la même permittivité relative et la même conductivité électrique.

Selon une première variante, la pièce comprend un organe de support et ledit au moins un élément d'extension est une couche déposée sur une surface de l'organe de support, un puits étant réalisé dans ladite couche pour former la cavité de logement de l'objet. A titre d'exemple, l'organe de support peut être une plaque, par exemple une lame de verre pour microscope, ou un conteneur, par exemple une boîte de Petri.

Selon une seconde variante, ledit au moins un élément d'extension est une couche, un puits non débouchant étant réalisé dans ladite couche pour former la cavité de logement de l'objet. Cette couche peut éventuellement être déposée sur une surface d'un organe de support (plaque ou boîte de Pétri).

Selon une troisième variante, ledit au moins un élément d'extension est un bloc, comportant une cavité fermée qui forme la cavité de logement de l'objet.

Selon une quatrième variante, la pièce comprend un organe de support creux ayant deux extrémités opposées reliées par une paroi et ledit au moins un élément d'extension est une première couche obturant l'une des deux extrémités de l'organe de support creux et une deuxième couche obturant l'autre des deux extrémités de l'organe de support creux, l'espace délimité par la paroi et les première et deuxième couches formant la cavité de logement de l'objet. Le support peut par exemple être un tube de type Eppendorf™. De préférence, les première et deuxième couches sont en un même matériau.

De préférence, la cavité de logement de l'objet dans les variantes ci-dessus est un cylindre ayant une hauteur plus ou moins importante (boîte de Petri, tube Eppendorf™, etc.). Les formes cylindriques sont couramment utilisées par les biologistes (boîtes de Pétri, tubes d'Eppendorf™, etc.). La géométrie cylindrique du puits présente l'avantage d'éviter les renforcements de champs sur les parties anguleuses que pourraient présenter une géométrie moins régulière.

Selon des modes de réalisation particuliers, la longueur d'un élément d'extension, de part et d'autre de la cavité, est au moins égale à la moitié de, de préférence au moins égale à la longueur de, et préférentiellement au moins égale à deux fois, la longueur de la cavité selon la direction de traversée.

Selon la revendication 1, l'étape de préparation de l'objet à tester comprend en outre la mise en place de la pièce dans un système d'exposition ayant un élément creux de conduction électrique en un matériau électriquement conducteur s'étendant selon une direction longitudinale et présentant, selon un plan de coupe passant par la direction longitudinale, deux portions électriquement conductrices en regard, la pièce étant disposée dans l'élément creux entre les deux portions électriquement conductrices de l'élément creux. De préférence, la pièce est disposée de manière à ce que la direction de traversée de la cavité de la pièce soit perpendiculaire à la direction longitudinale de l'élément creux. Le système d'exposition peut par exemple être un guide d'onde de section carrée, rectangulaire ou cylindrique.

Selon la revendication 2, l'étape de préparation de l'objet à tester comprend en outre la mise en place de la pièce dans un système d'exposition ayant au moins deux éléments de conduction électrique en matériau électriquement conducteur et disposés sensiblement parallèles (les deux éléments de conduction électrique pouvant être obliques, mais étant de préférence parallèles) et en regard, de manière à ce que la pièce soit disposée entre les deux éléments de conduction électrique. De préférence, la pièce est disposée de manière à ce que la direction de traversée de la cavité de la pièce soit perpendiculaire aux deux éléments de conduction électrique. Le système d'exposition peut par exemple être une ligne de transmission ayant au moins deux conducteurs électriques, par exemple une cellule TEM.

Dans les deux modes de réalisation, correspondant au deux revendications indépendantes, l'ensemble formé par l'objet à tester, ledit au moins un élément d'extension et éventuellement l'organe de support, s'il est présent, est installé respectivement entre deux portions électriquement conductrices ou entre les deux éléments de conduction électrique. De préférence, ledit au moins un élément d'extension de la pièce est en contact respectivement avec l'une des deux portions électriquement conductrices, préférentiellement avec les deux portions électriquement conductrices, ou avec l'un des deux éléments de conduction électrique, préférentiellement avec les deux éléments de conduction électrique. Les configurations dans lesquelles le matériau d'extension est en contact avec les deux portions électriquement conductrices ou avec les deux éléments électriquement conducteurs évitent d'avoir une répartition inhomogène du champ électrique entre le matériau d'extension et l'espace vide. Ces configurations assurent une homogénéité et une intensité maximale du champ électrique dans l'objet sous test. En d'autres termes, ces configurations dans lesquelles l'élément d'extension est en contact avec les deux conducteurs constitutifs d'une structure d'onde guidée (par exemple une ligne bifilaire, une cellule TEM, etc.) permettent de garantir un facteur de couplage maximal du champ électrique avec l'échantillon sous test, ainsi qu'une parfaite homogénéité du champ électrique dans cet échantillon. Dans les dispositifs de l'art antérieur, un espace d'air est présent entre l'échantillon et les conducteurs électriques. C'est par exemple le cas de la situation classique d'une boîte de Petri disposée entre les deux plaques électriquement conductrices d'une cellule TEM. Or, la présence de cet espace d'air entre l'échantillon et les conducteurs électriques se traduit par un renforcement du champ électrique dans cet espace d'air, au détriment du champ électrique couplé dans l'échantillon. Cela s'explique par l'écart important de permittivités diélectriques entre celle de l'échantillon (généralement une solution liquide aqueuse) et celle de l'air. Le facteur de couplage s'en trouve fortement réduit et le champ électrique présente un fort gradient à l'interface solution/air. On voit là tout l'intérêt qu'il y a à ce qu'il y ait un contact entre l'élément d'extension et au moins l'un des contacts conducteurs, de préférence entre l'élément d'extension et les deux contacts conducteurs.

La présente invention consiste à modifier artificiellement la géométrie de l'objet sous test en prolongeant ses dimensions dans au moins une direction (celle du champ électrique incident) en plaçant l'objet à tester en contact avec un matériau de caractéristiques électriques (permittivité relative et conductivité électrique) proches des siennes. Cela permet de maximiser le taux de couplage du champ électrique induit dans le volume de l'objet sous test avec le champ électrique incident, ce qui a pour effet d'augmenter l'intensité du champ électrique induit ; cela permet également d'augmenter l'homogénéité du champ électrique induit dans le volume de l'objet sous test.

La présente invention peut être appliquée au domaine du bio-électromagnétisme. Elle peut en particulier être utilisée au cours d'expérimentations qui mettent en oeuvre des objets biologiques ou chimiques et est particulièrement adaptée aux objets sous test sous forme liquide.

L'invention peut être utilisée au cours d'expérimentations d'illumination en espace libre, devant une antenne rayonnante ou bien encore à l'intérieur de certains systèmes d'exposition connus (telles que les cellules TEM, par exemple).

Il est à noter que la détermination du champ électrique induit dans l'objet sous test peut être facilitée grâce au procédé selon l'invention. La mesure du champ électrique induit est possible moyennant l'utilisation d'un capteur approprié, qui serait positionné dans le volume de l'objet à tester. Le positionnement du capteur dans le matériau d'extension à proximité de l'objet à tester permettrait d'obtenir une estimation du champ électrique induit, sans contact physique avec l'objet, ce qui est particulièrement avantageux (non pollution du capteur, non perturbation de l'objet sous test par le capteur).

D'autres avantages et caractéristiques de l'invention apparaîtront dans la description détaillée non limitative ci-dessous.

### BRÈVE DESCRIPTION DES DESSINS

Cette description sera faite au regard des dessins annexés parmi lesquels :
- les figures 1a et 1b représentent, respectivement selon une vue de côté en perspective et une vue de dessus, un premier mode de réalisation de l'invention, dans lequel la pièce comprend un organe de support qui est une boîte de Pétri ;
- les figures 2a et 2b représentent, respectivement selon une vue de côté en perspective et une vue en coupe longitudinale, un deuxième mode de réalisation possible de l'invention, dans lequel la pièce comprend un organe de support qui est une lame de microscope ;
- la figure 3 représente, selon une vue de côté en perspective, un troisième mode de réalisation possible de l'invention, dans lequel la pièce comprend un organe de support qui est une plaque à puits ;
- la figure 4 représente, selon une vue en coupe longitudinale, un quatrième mode de réalisation possible de l'invention, dans lequel la pièce ne comprend pas d'organe de support et l'élément d'extension est une couche dans laquelle est réalisé un puits non débouchant ;
- la figure 5 représente, selon une vue en perspective, un cinquième mode de réalisation possible de l'invention, dans lequel la pièce ne comprend pas d'organe de support et l'élément d'extension est un bloc dans lequel est réalisé un puits fermé ;
- la figure 6 représente un sixième mode de réalisation possible de l'invention, dans lequel la pièce comprend un organe de support qui est un tube de type Eppendorf™;
- la figure 7 représente un septième mode de réalisation possible de l'invention, dans lequel la pièce comprend un organe de support qui est un tube de type Eppendorf™ ayant une extrémité de forme conique ;
- les figures 8a et 8b représentent la cartographie des champs électriques crêtes (en valeur absolue) dans un plan de coupe horizontal passant par le centre de l'objet sous test, respectivement pour l'objet sous test dans un élément d'extension particulier (figure 8a) et pour l'objet sous test seul (figure 8b) ;
- les figures 9a et 9b représentent le profil associé relevé sur l'axe x (direction du champ électrique incident) dans l'objet sous test, respectivement pour l'objet sous test dans l'élément d'extension (figure 9a) et pour l'objet sous test seul (figure 9b) ;
- les figures 10a et 10b représentent les variations temporelles de la composante Ex (Ex étant la composante parallèle au champ électrique incident) au centre de l'objet sous test, respectivement pour l'objet sous test dans l'élément d'extension (figure 10a) et pour l'objet sous test seul (figure 10b).

### EXPOSÉ DÉTAILLÉ DE MODES DE RÉALISATION PARTICULIERS

Le principe sur lequel se base la présente invention consiste à modifier artificiellement la géométrie de l'objet à tester, de manière à prolonger artificiellement les dimensions de l'objet à tester à l'aide d'un matériau d'extension présentant des caractéristiques électriques proches de celles de l'objet.

Le matériau avec lequel l'extension de volume de l'OST est réalisée doit présenter une permittivité relative et, de préférence, une conductivité électrique les plus proches possibles de celles de l'objet à tester, afin de constituer un volume global le plus homogène possible. Le matériau pour le ou les éléments d'extension peut être un matériau composite, une céramique, un gel, etc.

L'ensemble formé par l'objet à tester et son extension volumique 10 se comporte alors comme un objet unique. Ainsi, le niveau du champ électrique induit dans l'objet sous test dépend des dimensions globales de l'ensemble « objet à tester + extension », et plus particulièrement de ses dimensions dans la direction du champ électrique. En effet, la répartition du champ électrique dépend de la géométrie globale, indépendamment de la géométrie de l'objet à tester. Dans la mesure où la dimension de l'objet à tester est faible vis-à-vis de la dimension globale de l'ensemble « objet à tester + extension » dans la direction du champ électrique incident (c'est la raison pour laquelle l'élément d'extension a une longueur d'au moins le tiers de la cavité selon la direction de traversée, de part à d'autre de la cavité dans laquelle est placée l'objet à tester), l'homogénéité et l'intensité du champ dans l'objet à tester est fortement améliorée par rapport à une configuration « objet à tester seul ».

En fait, la longueur totale du matériau d'extension dans la direction de traversée de la cavité (longueurs du ou des éléments d'extension de part et d'autre de la cavité) permet à la fois de s'affranchir de l'inhomogénéité de bord provoquée, dans l'art antérieur, par les parois du conteneur dans lequel est placé l'objet à tester et le vide environnant et, d'autre part, d'augmenter la longueur de couplage avec le champ électrique incident.

Au final, la longueur totale du matériau d'extension sera choisie en fonction du degré d'amélioration que l'on souhaite obtenir.

Par exemple, dans le cas illustré ci-dessous pour un tube Eppendorf™, comme la dimension de la cavité selon la direction de traversée est de quelques centimètres, le couplage entre le champ électrique incident et le champ électrique induit est déjà important. Le matériau d'extension sert alors principalement à l'homogénéisation du champ électrique induit et la longueur totale du matériau d'extension peut n'être que de 1/3 de la longueur de la cavité.

Au contraire, dans le cas illustré ci-dessous d'une cavité cylindrique de 4 mm de rayon réalisée dans une couche déposée dans une boîte de Petri, le matériau d'extension, en plus de son rôle d'homogénéisation, a également pour effet d'augmenter la longueur de couplage. La longueur totale du matériau d'extension doit donc être beaucoup plus importante que dans le cas précédent. Dans l'exemple illustré ci-dessous, on a par exemple un rapport supérieur à 10 entre la longueur de l'ensemble « objet sous test + matériau d'extension » dans la direction de traversée et la longueur de la cavité.

En tous les cas, la longueur optimale de couplage (l'ensemble « objet sous test + matériau d'extension ») étant assimilé à une antenne en réception) est une fraction de la longueur d'onde incidente. Si cette longueur est trop courte, le couplage sera peu amélioré ; mais si cette longueur est trop longue, on prend le risque d'avoir des ondes stationnaires au sein de l'ensemble « objet sous test + matériau d'extension ». C'est donc à l'homme du métier de régler la longueur du matériau d'extension en fonction des résultats qu'il souhaite obtenir.

Selon la configuration du ou des éléments d'extension, il est possible d'obtenir une extension du volume de l'objet à tester selon une direction (extension 1D) (voir l'exemple du tube Eppendorf™ ci-dessous), selon deux directions (extension 2D) (voir les exemples ci-dessous d'une plaque et d'une boîte de Petri) et selon trois dimensions (extension 3D).

La pièce peut comprendre un organe de support. Cet organe de support peut prendre diverses formes (boîte de Petri, plaque à puits, lame de microscope, tube Eppendorf™, etc.) et doit être adapté aux spécificités des expérimentations et à la nature des objets sous tests, qui définira la constitution du matériau d'extension (céramique, gel, etc.). Il est à noter que le choix de l'organe de support et du matériau d'extension se fait également en fonction de diverses contraintes de géométrie, de propriétés électriques, de propriétés mécaniques et chimiques (comme par exemple la résistance à la stérilisation, si l'organe de support et le ou les éléments d'extension sont destinés à être réutilisés).

Dans les exemples qui vont suivre, l'objet à tester est une suspension de 12,5 µL comprenant des Vésicules Uni lamellaires Géantes (GUV) dans une solution de 1,2-dioléoyl-sn-glycéro-3-phosphocholine (DOPC). La permittivité relative de l'objet est de 73,75 et sa conductivité électrique est de 1,73 S/m à la fréquence de 1,5 Ghz. Le ou les éléments d'extension sont en agar agar, qui présente une permittivité relative de 76 et une conductivité électrique de 0,37 S/m à cette même fréquence.

Selon un premier mode de réalisation représenté dans les figures la et 1b, la pièce 1 comprend un organe de support 6, qui est une boîte de Petri et l'élément d'extension 4 est une couche d'agar-agar, déposée dans la boîte de Petri, dans laquelle est réalisé un puits qui va former la cavité 2 de logement de l'objet à tester 3. De préférence, le puits est réalisé dans la couche au centre de la boîte de Petri, afin de maximiser la longueur de l'élément d'extension de part et d'autre de la cavité. Ainsi, dans cette représentation, la couche délimite la cavité selon la direction de traversée 5 sur l'ensemble de la paroi latérale cylindrique de la cavité (dans le plan de la couche) La paroi circulaire formant de fond de la cavité est formé par l'organe de support (la boîte de Pétri). Le champ électrique incident peut donc indifféremment être dirigé dans le plan de la couche. La contrainte sur la direction du champ électrique est alors éliminée.

Cette géométrie devient compatible avec l'utilisation d'une onde électromagnétique incidente à polarisation circulaire ou avec une onde incidente à polarisation elliptique, pour lesquelles la direction du champ électrique tourne dans un plan parallèle à la surface du fond de la boîte de Petri.

Au final, dans le cas de l'utilisation d'une boîte de Petri, il est possible de réduire le volume de l'objet à tester à celui d'un disque de quelques millimètres d'épaisseur disposé au centre d'un second disque évidé constitué par une couche en un matériau de caractéristiques diélectriques proches de celles de l'objet à tester.

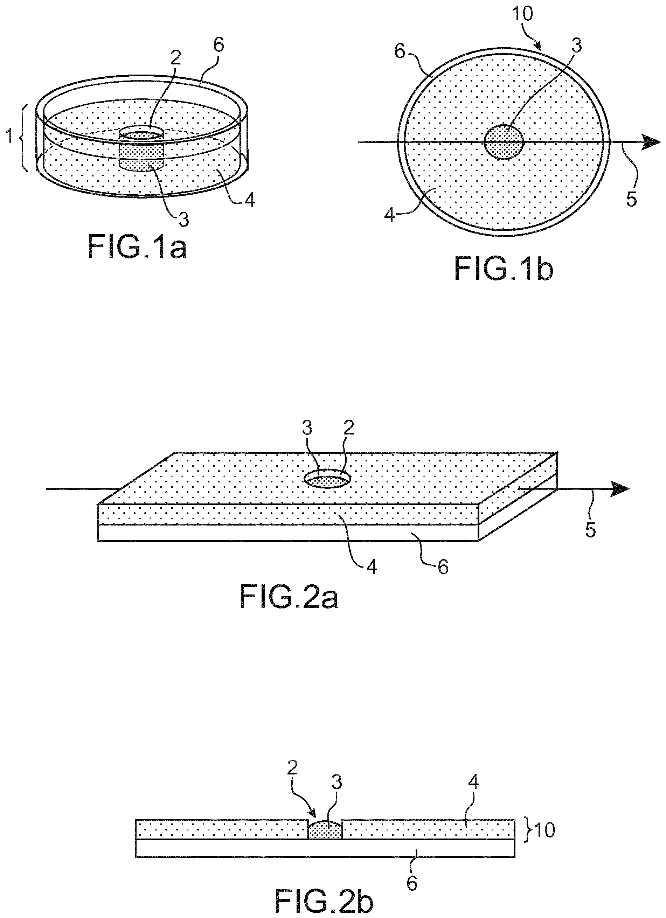

Selon un second mode de réalisation illustré dans les figures 2a et 2b, la pièce 1 comprend un organe de support 6, qui est une lame de verre standard pour microscope (24x80 mm), sur laquelle est déposée une couche de 1 mm (épaisseur non critique) d'agar agar. La couche comporte en son centre un puits cylindrique, qui constitue la cavité 2 pour l'objet à tester 3.

Il est possible de rajouter une lame de verre au-dessus de la couche d'agar agar, de manière à assurer l'étanchéité du puits et permettre un positionnement vertical de la pièce, lors de l'illumination par une onde à incidence horizontale, ou un positionnement « tête en bas », lors d'observations sous microscope.

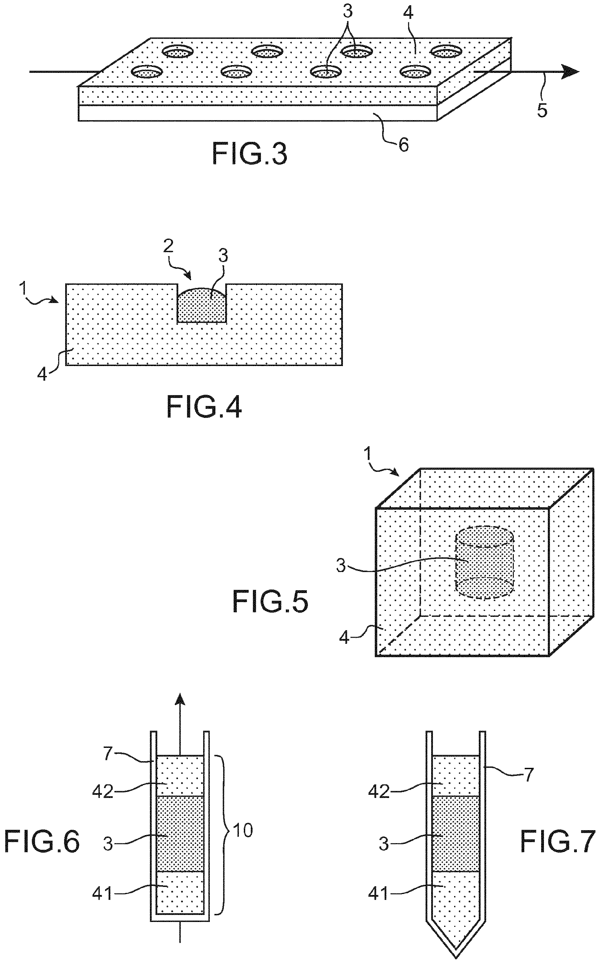

Il est possible de réaliser plusieurs puits dans cette couche, comme illustré dans la figure 3 qui représente un troisième mode de réalisation de l'invention. Cette réalisation permet de tester simultanément plusieurs objets à tester.

Il est à noter que le ou les puits peuvent être réalisés dans tout ou partie de l'épaisseur de la couche en matériau d'extension. Si la couche en matériau d'extension est disposée sur un organe de support, le ou les puits peuvent être des puits débouchants et traverser l'épaisseur de la couche. Dans la figure 4 est représenté un quatrième mode de réalisation, qui est le cas d'un puits non débouchant réalisé dans une couche épaisse en matériau d'extension. Cette configuration permet de se passer d'organe de support, le volume d'extension assurant la double fonction de cavité et de support. Selon un cinquième mode de réalisation, il est également possible de réaliser une cavité fermée à l'intérieur d'un bloc en matériau d'extension, comme représenté dans la figure 5. On a alors une extension du volume de l'objet dans les trois dimensions.

Selon un sixième mode de réalisation de l'invention représenté dans la figure 6, la pièce 1 comprend un organe de support creux, qui est un tube cylindrique, tel qu'un tube de type Eppendorf™, et l'élément d'extension est constitué de deux bouchons 41, 42 en agar agar, placés aux deux extrémités du tube, l'objet à tester 3 étant destiné à être placé dans le tube entre ces deux bouchons. En cas d'utilisation d'un tube de type Eppendorf™ conique (figure 7), cette solution présente l'avantage de s'affranchir de l'inhomogénéité supplémentaire apportée à l'intérieur du tube par cette géométrie particulière.

Pour illustrer l'efficacité du procédé objet de l'invention, nous avons réalisé deux simulations numériques à l'aide du logiciel CST Microwave studio™ en prenant, comme échantillon de référence, un objet à tester seul et le même objet disposé dans un puits réalisé dans une couche d'agar agar sur une lame pour microscope, comme illustré dans la figure 2.

La dimension du matériau d'extension dans la direction d'incidence du champ électrique règle l'amplitude du champ électrique induit dans l'objet à tester.

Par exemple, pour un objet à tester ayant la forme d'un disque de 4 mm de diamètre et une épaisseur de 1 mm, une permittivité relative (εᵣ) de 80, une conductivité électrique (σ) de 1,5 S/m, à une fréquence de 1,5 GHz, et un matériau d'extension virtuel (mais qui pourrait être une céramique à faibles pertes) de 24 mm × 80 mm × 1 mm ayant, à cette fréquence, une permittivité relative de 60 et une conductivité électrique nulle.

La comparaison des résultats obtenus pour ces deux simulations numériques montrent l'efficacité du procédé objet de l'invention sur l'amélioration de l'homogénéité du champ et de son intensité.

En effet, on constate que le rapport entre l'intensité du champ électrique induit maximale au centre de l'objet sous test et l'intensité du champ électrique incident varie de 10% (sans matériau d'extension) à 100% (avec un matériau d'extension). Le taux d'inhomogénéité, quant à lui, varie de 800% (sans matériau d'extension) à 14 % (avec matériau d'extension).

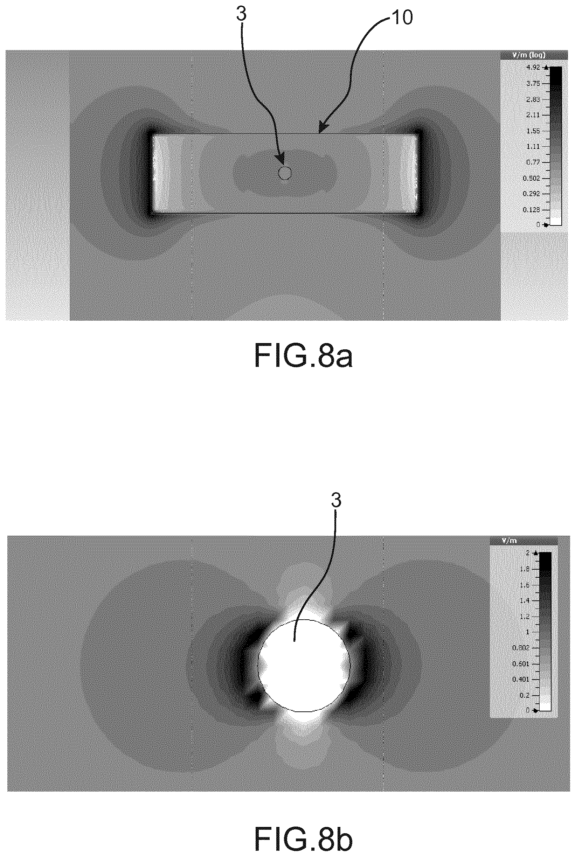

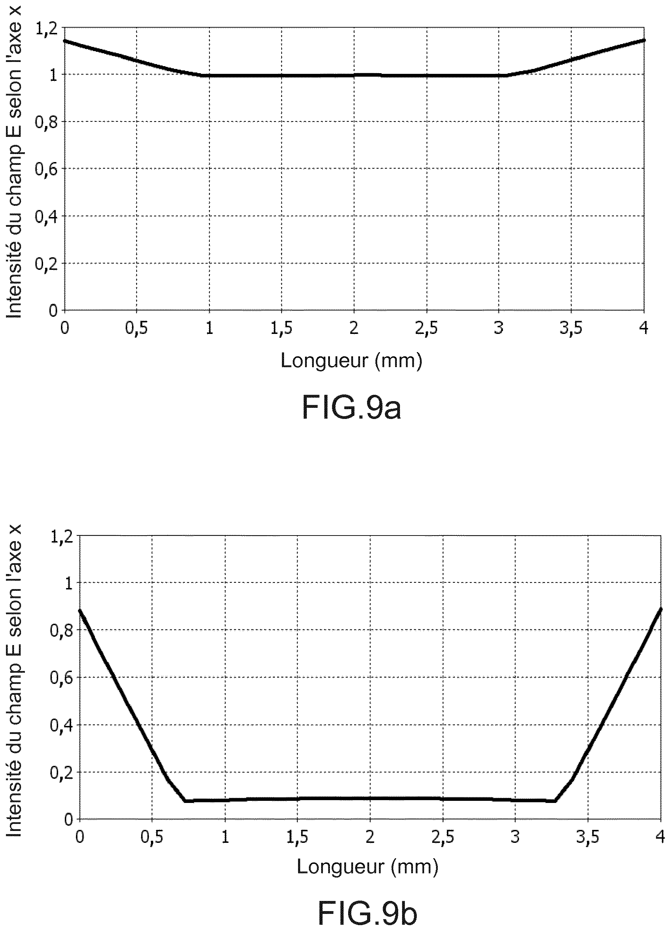

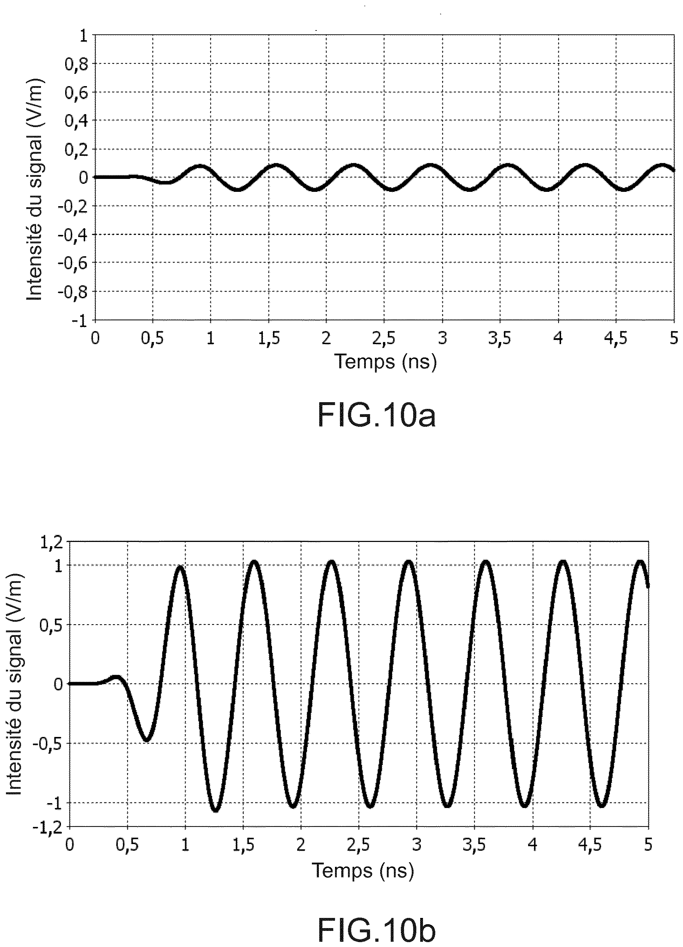

A titre d'illustration, nous donnons dans les figures 8a et 8b, 9a et 9b, 10a et 10b, les résultats obtenus en utilisant une onde électromagnétique plane à incidence normale, respectivement pour la configuration avec matériau d'extension (figures 8a, 9a et 10a) et pour la configuration sans matériau d'extension (figures 8b, 9b et 10b).

On constate que les gains avec l'utilisation d'un matériau d'extension sont supérieurs à un ordre de grandeur, tant au niveau de l'intensité du champ électrique induit qu'au niveau de l'homogénéité du champ électrique dans le volume de l'objet sous test et ce, en dépit des différences sensibles de permittivité et de conductibilité de l'objet sous test et du matériau d'extension.

Une simulation numérique complémentaire a été réalisée en utilisant les caractéristiques de l'agar agar 2% pour le matériau d'extension (permittivité relative de 76 et conductivité électrique de 0,37 S/m à une fréquence de 1,5 GHz). Les résultats obtenus montrent un taux d'inhomogénéité de 5%, au lieu des 14% obtenus avec le matériau d'extension virtuel considéré au cours de la précédente simulation. Ainsi, on constate qu'on améliore encore plus l'homogénéité et l'intensité du champ induit dans l'objet sous test lorsqu'on utilise un matériau d'extension dont la permittivité relative et la conductivité électrique sont proches de celles de l'objet sous test.

## Revendications

1. Procédé d'amélioration de l'homogénéité et de l'intensité du champ électrique induit dans un objet à tester (3) illuminé par une onde électromagnétique incidente, l'objet présentant une permittivité relative donnée, lequel procédé comprend :
- la préparation de l'objet à tester (3), comprenant les opérations de :
• fourniture d'une pièce (1) comprenant une cavité (2) pour le logement de l'objet (3) et au moins un élément d'extension (4) en un matériau présentant une permittivité relative égale à la permittivité relative de l'objet à +/- 50 % près, ledit au moins un élément d'extension (4) délimitant au moins en partie la cavité (2) et s'étendant de part et d'autre de la cavité (2) selon une direction qui est dite direction de traversée (5) de la cavité, sur une longueur qui est au moins égale, de part et d'autre de la cavité, au tiers de la longueur de la cavité selon la direction de traversée ;
• mise en place de l'objet (3) dans la cavité (2), de manière à ce que l'objet soit en contact avec ledit au moins un élément d'extension selon la direction de traversée ; et
• mise en place de la pièce (1) dans un système d'exposition ayant un élément creux de conduction électrique en un matériau électriquement conducteur s'étendant selon une direction longitudinale et présentant, selon un plan de coupe passant par la direction longitudinale, deux portions électriquement conductrices en regard, la pièce étant disposée dans l'élément creux entre les deux portions électriquement conductrices de l'élément creux ;
- l'application d'un champ électrique incident sur l'objet à tester par illumination de l'objet avec une onde électromagnétique incidente, la direction du champ électrique incident de l'onde électromagnétique incidente étant choisie identique à la direction de traversée (5) de la cavité (2).

2. Procédé d'amélioration de l'homogénéité et de l'intensité du champ électrique induit dans un objet à tester (3) illuminé par une onde électromagnétique incidente, l'objet présentant une permittivité relative donnée, lequel procédé comprend :
- la préparation de l'objet à tester (3), comprenant les opérations de :
• fourniture d'une pièce (1) comprenant une cavité (2) pour le logement de l'objet (3) et au moins un élément d'extension (4) en un matériau présentant une permittivité relative égale à la permittivité relative de l'objet à +/- 50 % près, ledit au moins un élément d'extension (4) délimitant au moins en partie la cavité (2) et s'étendant de part et d'autre de la cavité (2) selon une direction qui est dite direction de traversée (5) de la cavité, sur une longueur qui est au moins égale, de part et d'autre de la cavité, au tiers de la longueur de la cavité selon la direction de traversée ;
• mise en place de l'objet (3) dans la cavité (2), de manière à ce que l'objet soit en contact avec ledit au moins un élément d'extension selon la direction de traversée ; et
• mise en place de la pièce (1) dans un système d'exposition ayant au moins deux éléments de conduction électrique en matériau électriquement conducteur et disposés sensiblement parallèles et en regard, de manière à ce que la pièce soit disposée entre les deux éléments de conduction électrique ;
- l'application d'un champ électrique incident sur l'objet à tester par illumination de l'objet avec une onde électromagnétique incidente, la direction du champ électrique incident de l'onde électromagnétique incidente étant choisie identique à la direction de traversée (5) de la cavité (2).

3. Procédé selon la revendication 1 ou 2, dans lequel ledit au moins un élément d'extension de la pièce (1) est en contact avec au moins l'une des deux portions électriquement conductrices ou avec au moins l'un des deux éléments de conduction électrique.

4. Procédé selon l'une quelconque des revendications 1 à 3, dans lequel un élément d'extension a une permittivité relative égale à la permittivité relative de l'objet à +/-40 % près, de préférence à +/- 30 % près, de préférence encore à +/- 25% près, préférentiellement à +/- 20% près, et encore plus préférentiellement à +/- 10% près.

5. Procédé selon la revendication 4, dans lequel un élément d'extension a une permittivité relative égale à la permittivité relative de l'objet.

6. Procédé selon l'une quelconque des revendications 1 à 5, dans lequel l'objet à tester a une conductivité électrique donnée et ledit au moins un élément d'extension a une conductivité électrique égale à celle de l'objet à +/- 30% près, de préférence à +/- 25% près, préférentiellement à +/- 20% près, et encore plus préférentiellement à +/- 10% près.

7. Procédé selon l'une quelconque des revendications 1 à 6, dans lequel la pièce (1) comprend un organe de support (6) et ledit au moins un élément d'extension est une couche déposée sur une surface de l'organe de support, un puits étant réalisé dans ladite couche pour former la cavité (2) de logement de l'objet.

8. Procédé selon l'une quelconque des revendications 1 à 7, dans lequel ledit au moins un élément d'extension est une couche, un puits non débouchant étant réalisé dans ladite couche pour former la cavité (2) de logement de l'objet.

9. Procédé selon l'une quelconque des revendications 1 à 6, dans lequel ledit au moins un élément d'extension est un bloc, comportant une cavité fermée qui forme la cavité de logement de l'objet.

10. Procédé selon l'une quelconque des revendications 1 à 6, dans lequel la pièce (1) comprend un organe de support creux (7) ayant deux extrémités opposées reliées par une paroi et ledit au moins un élément d'extension est une première couche (41) obturant l'une des deux extrémités de l'organe de support creux et une deuxième couche (42) obturant l'autre des deux extrémités de l'organe de support creux, l'espace délimité par la paroi et les première et deuxième couches formant la cavité (2) de logement de l'objet.

11. Procédé selon l'une quelconque des revendications 1 à 6, dans lequel la longueur d'un élément d'extension, de part et d'autre de la cavité, est au moins égale à la moitié de, de préférence au moins égale à la longueur de, et préférentiellement au moins égale à deux fois, la longueur de la cavité selon la direction de traversée.

## Patentansprüche

1. Verfahren zum Verbessern der Homogenität und der Intensität eines elektrischen Feldes, das in einem zu prüfenden Objekt (3), das mit einer auftreffenden elektromagnetischen Welle bestrahlt wird, induziert wird, wobei das Objekt eine gegebene relative Permittivität aufweist, wobei das Verfahren Folgendes umfasst:
- Vorbereiten des zu prüfenden Objekts (3), mit den folgenden Schritten:
• Bereitstellen eines Teils (1), das einen Hohlraum (2) für die Aufnahme des Objekts (3) und wenigstens ein Verlängerungselement (4) aus einem Material, das eine relative Permittivität aufweist, die bis auf ±50 % gleich der relativen Permittivität des Objekts ist, umfasst, wobei das wenigstens eine Verlängerungselement (4) wenigstens teilweise den Hohlraum (2) begrenzt und sich beiderseits des Hohlraums (2) in einer Richtung erstreckt, die gleich der Durchgangsrichtung des Hohlraums ist;
• Anordnen des Objekts (3) in dem Hohlraum (2) in der Weise, dass das Objekt mit dem wenigstens einen Verlängerungselement in der Durchgangsrichtung in Kontakt ist; und
• Anordnen des Teils (1) in einem Beaufschlagungssystem, das ein stromführendes, hohles Element aus einem elektrisch leitenden Material besitzt, das in einer Längsrichtung verläuft und das in einer Schnittebene durch die Längsrichtung zwei einander gegenüberliegende elektrisch leitende Abschnitte aufweist, wobei das Teil in dem hohlen Element zwischen den beiden elektrisch leitenden Abschnitten des hohlen Elements angeordnet ist;
- Anlegen eines auf das zu prüfende Objekt auftreffenden elektrischen Feldes durch Bestrahlen des Objekts mit einer auftreffenden elektromagnetischen Welle, wobei die Richtung des auftreffenden elektrischen Feldes der auftreffenden elektromagnetischen Welle gleich der Durchgangsrichtung (5) des Hohlraums (2) gewählt wird.

2. Verfahren zum Verbessern der Homogenität und der Intensität des elektrischen Feldes, das in einem zu prüfenden Objekt (3), das mit einer auftreffenden elektromagnetischen Welle bestrahlt wird, induziert wird, wobei das Objekt eine gegebene relative Permittivität aufweist, wobei das Verfahren Folgendes umfasst:
- Vorbereiten des zu prüfenden Objekts (3), mit den folgenden Schritten:
• Bereitstellen eines Teils (1), das einen Hohlraum (2) für die Aufnahme des Objekts (3) und wenigstens ein Verlängerungselement (4) aus einem Material, das eine relative Permittivität aufweist, die bis auf ±50 % gleich der relativen Permittivität des Objekts ist, umfasst, wobei das wenigstens eine Verlängerungselement (4) wenigstens teilweise den Hohlraum (2) begrenzt und sich beiderseits des Hohlraums (2) in einer Richtung, die gleich der Durchgangsrichtung (5) des Hohlraums ist, über eine Länge erstreckt, die beiderseits des Hohlraums wenigstens gleich einem Drittel der Länge des Hohlraums in der Durchgangsrichtung ist;
• Anordnen des Objekts (3) in dem Hohlraum (2) in der Weise, dass das Objekt mit dem wenigstens einen Verlängerungselement in der Durchgangsrichtung in Kontakt ist; und
• Anordnen des Teils (1) in einem Beaufschlagungssystem, das wenigstens zwei stromführende Elemente aus einem elektrisch leitenden Material besitzt, die im Wesentlichen parallel und einander gegenüber angeordnet sind, derart, dass das Teil zwischen den beiden stromführenden Elementen angeordnet ist;
- Anlegen eines auf das zu prüfende Objekt auftreffenden elektrischen Feldes durch Bestrahlen des Objekts mit einer auftreffenden elektromagnetischen Welle, wobei die Richtung des auftreffenden elektrischen Feldes der auftreffenden elektromagnetischen Welle gleich der Durchgangsrichtung (5) des Hohlraums (2) gewählt wird.

3. Verfahren nach Anspruch 1 oder 2, wobei das wenigstens eine Verlängerungselement des Teils (1) mit wenigstens einem der beiden elektrisch leitenden Abschnitte oder mit wenigstens einem der beiden stromführenden Elemente in Kontakt ist.

4. Verfahren nach einem der Ansprüche 1 bis 3, wobei ein Verlängerungselement eine relative Permittivität besitzt, die bis auf ±40 %, vorzugsweise bis auf ±30 %, stärker bevorzugt bis auf ±25 %, noch stärker bevorzugt bis auf ±20 % und nochmals stärker bevorzugt bis auf ±10 % gleich der relativen Permittivität des Objekts ist.

5. Verfahren nach Anspruch 4, wobei ein Verlängerungselement eine relative Permittivität besitzt, die gleich der relativen Permittivität des Objekts ist.

6. Verfahren nach einem der Ansprüche 1 bis 5, wobei das zu prüfende Objekt eine gegebene spezifische elektrische Leitfähigkeit besitzt, die bis auf ±30 %, vorzugsweise bis auf ±25 %, stärker bevorzugt bis auf ±20 % und noch stärker bevorzugt bis auf ±10 % gleich jener des Objekts ist.

7. Verfahren nach einem der Ansprüche 1 bis 6, wobei das Teil (1) ein Trägerorgan (6) aufweist und das wenigstens eine Verlängerungselement eine Schicht ist, die auf eine Oberfläche des Trägerorgans aufgebracht ist, wobei in der Schicht ein Schacht ausgebildet ist, um den Hohlraum (2) für die Aufnahme des Objekts zu bilden.

8. Verfahren nach einem der Ansprüche 1 bis 7, wobei das wenigstens eine Verlängerungselement eine Schicht ist, wobei in der Schicht ein nicht offener Schacht ausgebildet ist, um den Hohlraum (2) für die Aufnahme des Objekts zu bilden.

9. Verfahren nach einem der Ansprüche 1 bis 6, wobei das wenigstens eine Verlängerungselement ein Block ist, der einen geschlossenen Hohlraum aufweist, der den Hohlraum für die Aufnahme des Objekts bildet.

10. Verfahren nach einem der Ansprüche 1 bis 6, wobei das Teil (1) ein hohles Trägerorgan (7) umfasst, dass zwei gegenüberliegende Enden besitzt, die durch eine Wand verbunden sind, und wobei das wenigstens eine Verlängerungselement eine erste Schicht (41), die eines der beiden Enden des hohlen Trägerelements verschließt, und eine zweite Schicht (42), die das andere der beiden Enden des hohlen Trägerorgans verschließt, besitzt, wobei der Raum, der durch die Wand und die erste und die zweite Schicht begrenzt ist, den Hohlraum (2) für die Aufnahme des Objekts bildet.

11. Verfahren nach einem der Ansprüche 1 bis 6, wobei die Länge des Verlängerungselements beiderseits des Hohlraums wenigstens gleich der Hälfte der Länge, vorzugsweise wenigstens gleich der einfachen Länge und stärker bevorzugt wenigstens gleich der doppelten Länge des Hohlraums in der Durchgangsrichtung ist.

## Claims

1. A method for improving the uniformity and intensity of the electric field induced in an object to be tested (3) illuminated by an incident electromagnetic wave, the object having a given relative permittivity, wherein the method includes:
- preparation of the object to be tested (3), the preparation comprising:
• supply of a part (1) having a cavity (2) to house the object (3) and at least one extension element (4) made of a material with a relative permittivity equal to the relative permittivity of the object within +/- 50 %, said at least one extension element (4) partly delimiting the cavity (2) and extending on each side of the cavity (2) along a direction said to be the passage direction (5) of the cavity, over a length which is, on each side of the cavity, equal to at least one third of the length of the cavity along the passage direction; and
• placement of the object (3) in the cavity (2) such that the object is in contact with said at least one extension element along the passage direction;
• placement of the part (1) in an exposure system having a hollow electrically conducting element made of an electrically conducting material extending along a longitudinal direction and having two electrically conducting portions facing each other in a section plane along a longitudinal direction, the part being placed in the hollow element between the two electrically conducting portions of the hollow element;
- application of an incident electric field on the object to be tested by illumination of the object with an incident electromagnetic wave, the direction of the incident electric field of the incident electromagnetic wave being chosen to be identical to the passage direction (5) of the cavity (2).

2. A method for improving the uniformity and intensity of the electric field induced in an object to be tested (3) illuminated by an incident electromagnetic wave, the object having a given relative permittivity, wherein the method includes:
- preparation of the object to be tested (3), the preparation comprising:
• supply of a part (1) having a cavity (2) to house the object (3) and at least one extension element (4) made of a material with a relative permittivity equal to the relative permittivity of the object within +/- 50 %, said at least one extension element (4) partly delimiting the cavity (2) and extending on each side of the cavity (2) along a direction said to be the passage direction (5) of the cavity, over a length which is, on each side of the cavity, equal to at least one third of the length of the cavity along the passage direction; and
• placement of the object (3) in the cavity (2) such that the object is in contact with said at least one extension element along the passage direction;
• placement of the part (1) in an exposure system having at least two electrically conducting elements made of an electrically conducting material and placed approximately parallel to and facing each other, such that the part is placed between the two electrical conducting elements;
- application of an incident electric field on the object to be tested by illumination of the object with an incident electromagnetic wave, the direction of the incident electric field of the incident electromagnetic wave being chosen to be identical to the passage direction (5) of the cavity (2).

3. The method according to claim 1 or 2, wherein said at least one extension element of the part (1) is in contact with at least one of the two electrically conducting portions or with at least one of the two electrically conducting elements.

4. The method according to any one of claims 1 to 3, wherein an extension element has a relative permittivity equal to the relative permittivity of the object within +/-40%, preferably within +/- 30%, even more preferably within +/-25%, even more preferably within +/-20% and even more preferably within +/-10%.

5. The method according to claim 4, wherein the relative permittivity of an extension element is equal to the relative permittivity of the object.

6. The method according to any one of claims 1 to 5, wherein the object to be tested has a given electrical conductivity and the electrical conductivity of said at least one extension element is equal to that of the object within +/-30%, preferably within +/-25%, even more preferably within +/-20% and even more preferably within +/-10%.

7. The method according to any one of claims 1 to 6, wherein the part (1) comprises a support device (6) and said at least one extension element is a layer deposited on a surface of the support element, a well being made in said layer to form the cavity (2) in which the object is to be housed.

8. The method according to any one of claims 1 to 7, wherein said at least one extension element is a layer, a non-through well being made in said layer to form the cavity (2) in which the object is to be housed.

9. The method according to any one of claims 1 to 6, wherein said at least one extension element is a block comprising a closed cavity that forms the cavity in which the object is to be housed.

10. The method according to any one of claims 1 to 6, wherein the part (1) comprises a hollow support device (7) with two opposite ends connected by a wall and said at least one extension element is a first layer (41) closing off one of the two ends of the hollow support device and a second layer (42) closing off the other of the two ends of the hollow support device, the space delimited by the wall and the first and second layers forming the cavity (2) in which the object is to be housed.

11. The method according to any one of claims 1 to 6, wherein the length of an extension element on each side of the cavity is equal to at least half, and preferably equal to at least the length, and even more preferably equal to at least twice the length of the cavity along the passage direction.
